(19) Europäisches Patentamt
European Patent Office
Office européen des brevets

(11) Numéro de publication: **0 202 976**
**B1**

# (12) FASCICULE DE BREVET EUROPEEN

(45) Date de publication du fascicule du brevet: **13.07.88**

(51) Int. Cl.⁴: **C 07 C 127/15**

(21) Numéro de dépôt: **86400799.2**

(22) Date de dépôt: **15.04.86**

## (54) Nouveau procédé de préparation de dérivé acrylique de l'urée.

(30) Priorité: **23.04.85 FR 8506103**

(43) Date de publication de la demande: **26.11.86 Bulletin 86/48**

(45) Mention de la délivrance du brevet: **13.07.88 Bulletin 88/28**

(84) Etats contractants désignés: **AT BE CH DE GB IT LI LU NL SE**

(56) Documents cités:
**CH - A - 316 404**

**CHEMICAL ABSTRACTS, vol. 73, no. 19, novembre 1970, page 26, abstract no. 99558e, Columbus, Ohio, US**

(73) Titulaire: **NORSOLOR S.A., Tour Aurore Place des Reflets, F-92080 Paris la Defense Cédex 5 (FR)**

(72) Inventeur: **Garrigue, Roger, 23, rue du Capitaine Escudié, F-31000 Toulouse (FR)**
Inventeur: **Lalo, Jack, 21, rue de la Digue - Appt. 128, F-31300 Toulouse (FR)**

(74) Mandataire: **Rieux, Michel, NORSOLOR Service Propriété Industrielle Tour Aurore Place des Reflets Cédex no. 5, F-92080 Paris la Défense 2 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un nouveau procédé de préparation de dérivés acryliques de l'urée, elle a plus particulièrement un nouveau procédé de préparation de dérivé acrylique de l'urée obtenu en particulier à partir d'urée et d'acrylamide.

Certain dérivés acryliques de l'urée sont connus depuis longtemps. Les composés acryliques utilisés comme produits de départ sont le plus souvent l'acrylamide ou l'acrylonitrile.

C'est ainsi qu'on a proposé (voir brevet Suisse CIBA N° 316 404) la préparation de l'acrylamido-méthylène-urée selon un procédé en deux étapes qui consiste en faire réagir d'abord, dans une première étape en milieu fortement acide du paraformaldéhyde avec de l'urée, la température du milieu réactionel pouvant s'élever jusqu'à 70°C, puis dans une deuxième étape à additionner à basse température de l'acrylonitrile sur le produit de réaction obtenu dans la première étape. Après neutralisation du milieu réactionnel, les auteurs affirment obtenir un produit de point de fusion égale à 130-131 °C; le produit obtenu est décrit comme étant l'acrylamido-méthylène-urée dont la formule serait:

$CH_2=CH\text{-}C\text{-}NH\text{-}CH_2\text{-}NH\text{-}CO\text{-}NH_2$
||
O

Le procédé mis en œuvre dans le brevet suisse peut se résumer de la façon suivante:

*Ière étape*

Action de l'urée sur le formol en milieu sulfurique selon la réaction:

$NH_2\text{-}C\text{-}NH_2 + H\text{-}C\text{-}H \xrightarrow{H_2SO_4\ (98\%)} NH_2\text{-}C\text{-}NH\text{-}CH_2\ O\ H$
|| || ||
O O O

qui conduit à la monométhylolurée.

*2ème étape*

Action de l'acrylonitrile sur la monométhyloruréé en présence d'acide sulfurique selon la réaction

$CH_2=CH\text{-}C\equiv N + HO\text{-}CH_2\text{-}NH\text{-}C\text{-}NH_2 \xrightarrow{H_2SO_4\ (98\%)}$
||
O

$CH_2=CH\text{-}C\text{-}NH\text{-}CH_2\text{-}NH\text{-}C\text{-}NH_2$
|| ||
O O

qui devrait conduire à la formation d'acrylamido-méthylène-urée.

Le point de fusion est la seule caractéristique du produit acrylique décrit dans le brevet suisse précipité, aucun résultat analytique permettant de confirmer la structure du produit est indiqué. Afin de vérifier le bien fondé du procédé mis en œuvre, la demanderesse a réalisé des essais mettant en œuvre les conditions opératoires du brevet antérieur: on a alors obtenu par cette méthode un produit dont le point de fusion est de 131 °C, mais avec un rendement très faible voisin de 5% et dont la composition, déterminée par analyse chromatographique en couche mince révèle la présence de plusieurs produits parmi lesquels on identifie l'urée, le bisacrylamido-méthane, mais pas d'acrylamido-méthylène-urée. La même méthode analytique réalisée sur un échantillon neutralisé, mais dilué, contenant tous les produits de la réaction révèle bien effectivement la présence , mais en très faible quantité, d'acrylamido-méthylène-urée. En réalité le produit même s'il se forme en mettant en œuvre le mode opératoire décrit dans le brevet suisse a peu de chance d'être isolé par la méthode décrite en raison de sa faible solubilité dans l'eau (inférieure à 2% à 20°C) et de son insolubilité dans l'acétone.

Sans vouloir être lié par des mécanismes chimiques théoriques, la demanderesse pense que la mise en œuvre des conditions opératoires du brevet suisse conduit à un mélange de bisacrylamido-méthane et d'urée car il semble que la réaction de la Ière étape en milieu sulfurique urée + formol

O O O
|| || ||
$NH_2\text{-}C\text{-}NH_2 + H\text{-}C\text{-}H \leftrightharpoons NH_2\text{-}C\text{-}NH\text{-}CH_2\ OH$

est une réaction équilibrée dans laquelle en réalité le formol réagit dans la duexième étape avec l'acrylonitrile pour donner du bisacrylamido-méthane selon la réaction

$H_2O + H\text{-}C\text{-}H + 2\ CH_2=CH\text{-}C\equiv N \xrightarrow{H_2SO_4}$
||
O

$CH_2=CH\text{-}C\text{-}NH\text{-}CH_2\text{-}NH\text{-}C\text{-}CH=CH_2$
|| ||
O O

Le besoin se fait donc sentir de mettre au point un procédé permettant de fabriquer de véritables dérivés acryliques de l'urée à partir d'urée, de formol et d'acrylonitrile.

La présente invention concerne un procédé de préparation de dérivé acrylique de l'urée, de formule:

$CH_2=CH\text{-}C\text{-}NH\text{-}CH_2\text{-}NH\text{-}C\text{-}NH_2$
|| ||
O O

à partir d'urée, de formol et d'acrylamide caractérisé en ce qu'on fair réagir dans une première étape dans un solvant non réactif l'acrylamide avec le formol à pH basique au moins égal à 7 et en ce que dans une deuxième étape on fait réagir le produit de réaction obtenu dans la première étape sur l'urée à pH acide au plus égal à 4,5 la quantité d'urée mise en œuvre étant au moins égale à un équivalent molaire et de préférence supérieure à quatre équivalents molaires pour un équivalent calculé sur le produit de la réaction obtenue dans la première étape.

La mise en œuvre d'un tel procédé permet de préparer le dérivé acrylique de l'urée avec des rendements avoisinant 70% et dont la structure chimique est confirmée par les méthodes analytiques usuelles:

analyse élémentaire, spectre Infra-rouge et R.M.N. du proton et du $^{13}C$.

Le procédé de la présente invention consiste à faire réagir dans un solvant non réactif, l'acrylamide avec le formol à pH basique au moins égal à 7. Les solvants non réactifs qui conviennent pour la mise en œuvre du procédé de l'invention sont choisis parmi l'eau, le diméthylformamide; lorsqu'on utilise l'eau comme solvant le formol est introduit sous forme de solution aqueuse; lorsqu'on utilise un solvant organique, le formol est introduit sous forme de paraformaldéhyde. On ajuste le pH à une valeur au moins égale à 7 à l'aide , par exemple, d'amines aliphatiques tels que le triéthylamine pour des valeurs de pH inférieur à 7 on réduit la vitesse de formation du dérivé. Préférentiellement, cette première étape est conduite en présence d'un inhibiteur de polymérisation éther méthylique de l'hydroquinone par exemple. Cette première étape est conduite à une température qui dépend de la nature du solvant: si le solvant utilisé est de l'eau la température réactionnelle est voisine de 50°C, si le solvant mis en œuvre est du diméthyl formamide, la réaction est conduite à une température voisine de 80°C.

La duxième étape du procédé objet de l'invention consiste ensuite à faire réagir le produit obtenu lors de la première étape avec de l'urée à pH acide au plus égal à 4,5. On a en effet trouvé que la mise en œuvre du procédé réalisé à pH supérieur à 4,5 conduit à des taux de conversion voisin de zéro. Le pH est ajusté par exemple en utilisant de l'acide sulfurique concentré ou de l'acide chlorhydrique. La température réactionnelle de cette deuxième étape dépend aussi du solvant non réactif mis en œuvre; si le solvant est de l'eau, la réaction est conduite à température ambiante, si le solvant est un solvant organique la réaction est conduite à une température voisine de 80°C. La quantité d'urée mise en œuvre est au moins égale à un équivalent et de préférence supérieure à quatre équivalents molaires pour un équivalent calculé sur le produit de la réaction obtenu dans la première étape. On a en effet trouvé qu'il est préférable de mettre en œuvre quatre équivalents molaires si l'on veut éviter la formation de [NN'bis(acrylamido-méthylène)-urée] comme impureté.

Le produit obtenu selon le procédé, objet de l'invention, est polymérisable ou copolymérisable et condensable avec l'urée en présence de formol. Il convient particulièrement comme additif dans les aminoplastes et phénoplastes notamment ceux utilisés comme colles à bois; il peut aussi être utilisé comme agent réticulant.

Les exemples suivants illustrent la présente invention; les quantités sont exprimées en parties en poids:

*Exemple 1*

*Synthèse de l'acrylamido méthylène urée dans l'eau*

*1ère étape*

Dans un réacteur agité muni d'un condenseur à reflux, on introduit la charge suivante:

— 20 parties d'acrylamide
— 22,8 partie d'une solution aqueuse de formol à 37%
— 0,2 partie de triéthylamine
— 0,01 partie d'éther méthylique de l'hydroquinone.

On mantient le milieu réactionnel à 50°C pendant 1 heure.

*2ème étape*

Après refroidissement du milieu réactionnel et maintient à 10°C, on ajoute 67,6 parties d'urée et 15 parties d'acide chlorhydrique concentré. Le pH du milieu réactionnel est alors de 1. Le milieu réactionnel est agité pendant 15 heures à températures ambiante. Il se forme un précipité tout au long de la réaction. Après filtration et recristallisation dans l'eau, on obtient 27,5 parties d'acrylamide méthylène-urée. Le rendement de la réaction est de 67,5%.

Point de fusion: brunissement entre 265-270°C.

L'analyse élementaire donne les résultatis suivants:

| | Trouvé | Calculé |
|---|---|---|
| - C: | 41,71 | 41,95 |
| - H: | 6,28 | 6,29 |
| - N: | 29,24 | 29,37 |

Le spectre infra-rouge permet de confirmer la structure du produit obtenu (ci-joint en annexe figure 1).

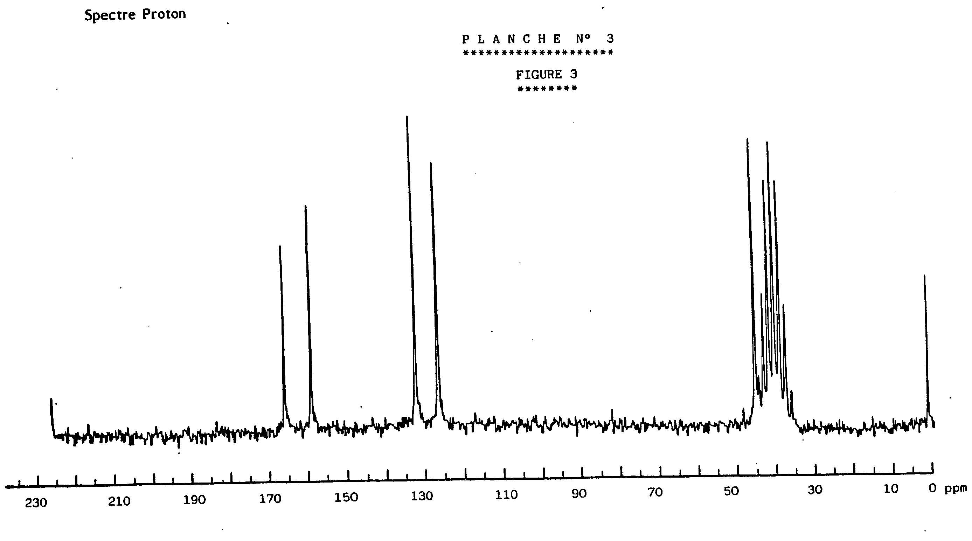

Le spectre RMN permet de confirmer la structure de l'acrylamido-méthylène-urée (ci-joint en annexe figures 2 et 3).

*Exemple 2*

*Synthèse de l'acrylamido-méthylène-urée dans un solvant organique*

*1ère étape*

Dans le réacteur utilisé dans l'exemple 1, on introduit la charge suivante:

— 20 parties d'acrylamide
— 200 parties de diméthylformamide
— 9 parties de paraformaldéhyde (pureté 94%)
— 0,4 partie de triéthylamine
— 0,010 partie d'éther méthylique de l'hydroquinone.

Le milieu réactionnel est chauffé 6 heures à 80%C.

*2ème étape*

Au milieu réactionnel de l'étape 1 et refroidi à 20°C, on ajoute

— 67,6 parties d'urée
— 1 partie d'acide sulfurique concentré.

On chauffe à 80°C pendant 2 heures. Après refroidissement, filtration et recristallisation, on obtient 8 parties d'un produit solide qui présente les caractéristiques suivantes:

Point de fusion: brunissement entre 265-270°C.
Analyse élémentaire:

| | Trouvé | Calculé |
|---|---|---|
| - C: | 41,63 | 41,95 |
| - H: | 6,19 | 6,29 |
| - N: | 29,30 | 29,37 |

Les spectres Infrarouge et R.M.N. sont identiques à ceux de l'exemple 1.

Ils confirment la préparation de l'acrylamido-méthylène-urée.

*Exemple 3*

Les essais suivants ont été réalisés en faisant réagir le produit de la réaction obtenu dans la première étape des exemples 1 ou 2 avec de l'urée: la réaction est conduite à différente température et à pH variable ainsi qu'en mettant en œuvre différents rapports molaires du produit de réaction obtenu dans l'étape 1, et de l'urée additionnée dans l'étape 2.

Le produit obtenu dans l'étape 1 a pour formule:

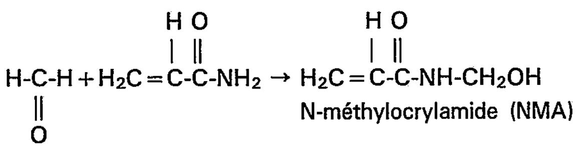

N-méthylocrylamide (NMA)

Le produit obtenu dans l'étape 2 est l'acrylamido-méthylène-urée obtenu selon la réaction

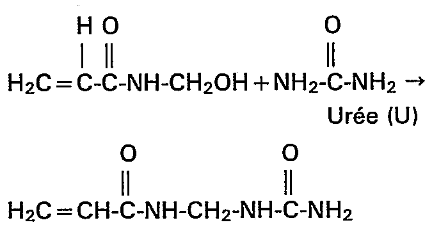

Urée (U)

AMU

Les résultats suivants ont été obtenus

| pH | $[U]_o/[NMA]_o$ | T° C | $[NMA_o]$ | τ % |
|---|---|---|---|---|
| 4,2 | 1 | 40 | 1,6 | 0 |
| 3,2 | 1 | 40 | 1,56 | 2 |
| 1,8 | 1 | 40 | 1,5 | 22 |
| 3,3 | 4 | 40 | 1,3 | 4 |
| 2,6 | 4 | 40 | 1,3 | 20 |
| 1,7 | 4 | 40 | 1,3 | 70 |
| 2,1 | 6 | 40 | 1,1 | 60 |
| 2,7 | 4 | 60 | 1,3 | 63 |

$[U]_o/[NMA]_o$ = rapport molaire $\dfrac{\text{urée départ}}{\text{N méthylolacrylamide départ}}$

τ % = taux de conversion au bout de 2 heures de réaction défini par la relation:

$$= [1 - \frac{[NMA]}{[NMA]_o}] \times 100$$

Le dosage des groupes méthylol a été réalisé en mettant en œuvre une méthode de dosage en retour iode-thiosulfate. Selon cette méthode on ajoute à une solution contenant 1 ml d'échantillon et 10 ml de soude 2 N, 50 ml d'une solution aqueuse d'iode $\frac{N}{10}$. L'iode en excès est dosé pour une solution aqueuse de thiosulfate $\frac{(N)}{10}$ après addition de 20 $cm^3$ d'acide sulfurique 2 N.

*Exemple 4* (Exemple comparatif)

A titre comparatif on a réalisé l'exemple 3 du brevet suisse 316 404 en mettant en œuvre les mêmes conditions opératoires.

On dissout 27 parties de paraformaldéhyde dans 180 parties d'acide sulfurique à 98%. Le mélange réactionnel est chauffé à 70°C puis refroidi à une température inférieure à 10°C.

On ajoute ensuite 54 parties d'urée. On maintient le milieu réactionnel à température ambiante pendant trois heures puis on additionne 47,7 parties d'acrylonitrile après avoir refroidi le milieu à une température de 15°C.

On maintient le mélange réactionnel à température ambiante pendant 12 heures puis on le neutralise par addition d'une solution de soude à 50% tout en refroidissant afin de maintenir le mélange à une température inférieure à 10°C.

Après neutralisation le sulfate de sodium formé précipite, il est alors filtré et lavé avec 400 parties d'acétone. Après élimination de l'eau sous vide à une température de 40°C on élimine le sulfate de sodium qui a précipité par filtration et lavage à l'acétone. Le filtrat aquex obtenu et l'acétone de lavage sont ensuite réunis et distillés. Il se forme alors un précipité que l'on sépare par filtration: on en receuille 6 parties. L'analyse par chromatographie sur couche mince permet de mettre en évidence la présence d'urée, de bisacrylamido méthane et de sulfate de sodium. Après refroidissement il se forme un précipité que l'on filtre: sa masse est de 5 parties, son point de fusion est de 131°C. Par chromatographie en couche mince on identifie de l'urée et du bisacrylamido-méthane. En prélevant un échantillon que l'on neutralise et dilue, on met en évidence des quantité infimes d'acrylamido-méthylène-urée.

*Exemple 5*

L'exemple 1 est répété en mettant en œuvre dans la pemière étape:

- 71 parties d'acrylamide
- 81 parties d'une solution de formol à 37%
- 2 parties de triethylamine
- 0,04 partie d'éther méthylique de l'hydroquinone;

dans la deuxième étape.

- 180 parties d'urée.

Après traitement on recueille 101 parties d'un produiti: le rendement de la réaction est de 70.6%.

L'analyse par chromatographie en couche mince

permet de mettre en évidence la présence de N,N' bis acrylamido-méthylène-urée.

*Exemple 6*

Une résine urée-formol présente les caractéristiques suivantes

| | |
|---|---|
| Viscosité à 20°C: | 5,5 poises |
| Extrait sec: | 65% |
| pH: | 8 |
| $\frac{F}{NH_2}$: | 0,55 |

Cette résine est modifiée par 6,5% d'acrylamido méthylène urée. On dispose alors d'une résine qui présente les caractéristiques suivantes:

| | |
|---|---|
| Viscosité à 20°C: | 3,8 poises |
| Extrait sec: | 65% |
| pH: | 8 |
| $\frac{F}{NH_2}$: | 0,55 |

Ces deus résines sont utilisées pour la fabrication de panneaux particules monocouches à partir de copeaux de bois en mettant en œuvre les mêmes conditions opératoires: les résines sont durcies à l'aide d'une solution de chlorure d'ammonium. Le tableau suivant indique les caractéristiques des panneaux ontenus:

| Type résine | Résine modifiée | Résine non modifiée |
|---|---|---|
| Résistance en traction (kg/cm²) | 6,1 | 6,3 |
| Gonflement à l'eau V 20, % | 12,1 | 12,4 |
| Absorption, % | 60 | 62 |
| Humidité panneau % | 7,4 | 7,3 |
| Formol perforateur mg/100 g | 10,5 | 12,5 |

Les mesures ont été déterminées selon les normes suivantes:

| | |
|---|---|
| — Teneur en formol (perforateur): | Norme EN 120 |
| — Epaisseur, masse volumique et humidité: | Norme DIN 52361 |
| — Traction V20: | Norme DIN 68763 |
| — Gonflement (%): | Norme DIN 52364 |

**Revendications**

1. Procédé de préparation de dérivé acrylique de l'urée, de formule:

$$CH_2=CH-\underset{\underset{O}{\|}}{C}-NH-CH_2-NH-\underset{\underset{O}{\|}}{C}-NH_2$$

à partir d'urée, de formol et d'acrylamide caractérisé en ce qu'on fait réagir dans une première étape dans un solvant non réactif l'acrylamide avec le formol à pH basique au moins égal à 7 et en ce que dans une deuxième étape on fait réagir le produit de réaction obtenu dans la première étape sur l'urée à pH acide au plus égal à 4,5 la quantité d'urée mise en œuvre étant au moins égale à un équivalent molaire et de préférence supérieure à quatre équivalents molaires pour un équivalent calculé sur le produit de la réaction obtenue dans la première étape.

2. Procédé selon 1 caractérisé en ce que le solvant non réactif est choisi parmi l'eau et le diméthylformamide.

3. Procédé selon 1 et 2 selon lequel la réaction est conduite à une température comprise entre 50 et 80°C.

**Patentansprüche**

1. Verfahren zur Herstellung einer Harnstoffacrylverbindung der Formel

$$CH_2=CH-\underset{\underset{O}{\|}}{C}-NH-CH_2-NH-\underset{\underset{O}{\|}}{C}-NH_2$$

aus Harnstoff, Formaldehyd und Acrylamid, dadurch gekennzeichnet, dass man in einem ersten Schritt in einem nicht reaktiven Lösungsmittel Acrylamid mit Formaldehyd bei einem basischen pH-Wert von mindestens 7 reagieren lässt und, und dass man in einem zweiten Schritt das im ersten Schritt erhaltene Reaktionsprodukt auf Harnstoff bei einem sauren pH-Wert von höchstens 4,5 einwirken lässt, wobei die eingesetzte Harnstoffmenge mindestens 1 Moläquivalent, vorzugsweise mehr als 4 Moläquivalente, auf 1 berechnetes Äquivalent des im ersten Schritt erhaltenen Reaktionsproduktes beträgt.

2. Verfahren nach Anspruch 1, dadurch gekennzeichnet, dass das nicht reaktive Lösungsmittel Wasser oder Dimethylformamid ist.

3. Verfahren nach Anspruch 1 und 2, bei welchem die Reaktion bei einer Temperatur zwischen 50° und 80° durchgeführt wird.

**Claims**

1. Method for preparing an acrylic derivative of urea, of formula:

$$CH_2=CH-\underset{\underset{O}{\|}}{C}-NH-CH_2-NH-\underset{\underset{O}{\|}}{C}-NH_2$$

from urea, formaldehyde and acrylamide, characterized in that, in a first stage, acrylamide is reacted with formaldehyde in an unreactive solvent at a basic pH of at least 7 and in that, in a second stage, the reaction product obtained in the first stage is reacted with urea at an acidic pH not exceeding 4.5, the

quantity of urea employed being at least one molar equivalent and preferably more than four molar equivalents for one equivalent calculated on the product of the reaction obtained in the first stage.

2. Method according to 1, characterized in that the unreactive solvent is chosen from water and dimethylformamide.

3. Method according to 1 and 2, according to which the reaction is carried out at a temperature of between 50 and 80°C.

PLANCHE N° 1
************************

FIGURE 1
********

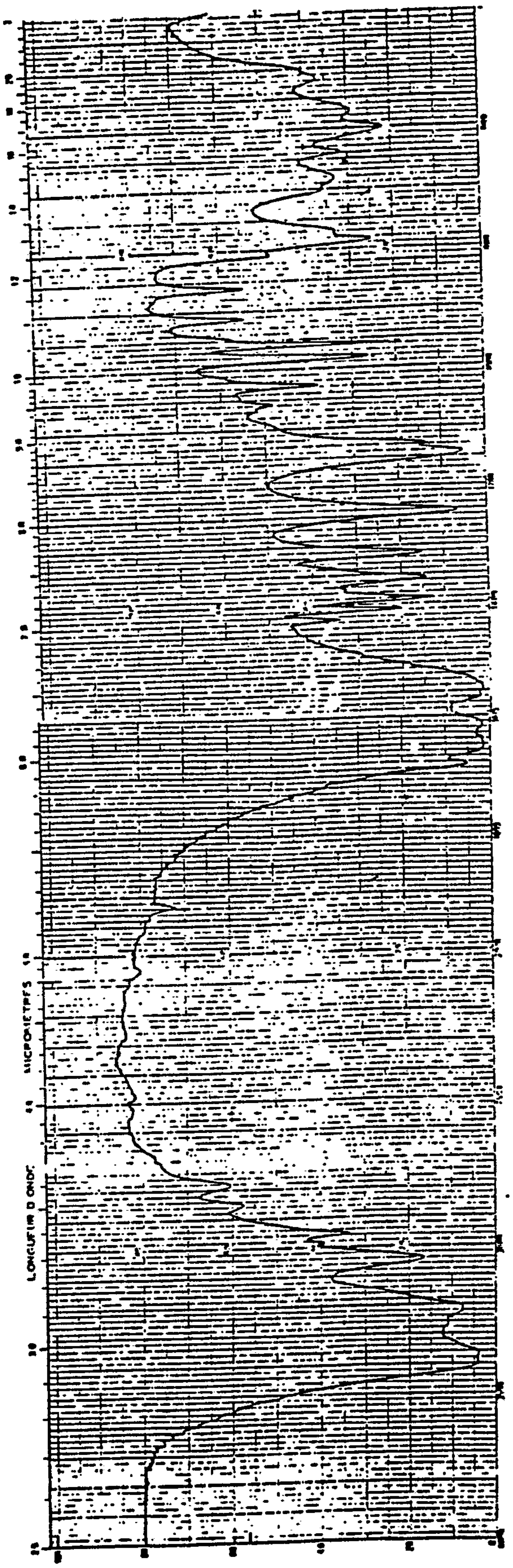

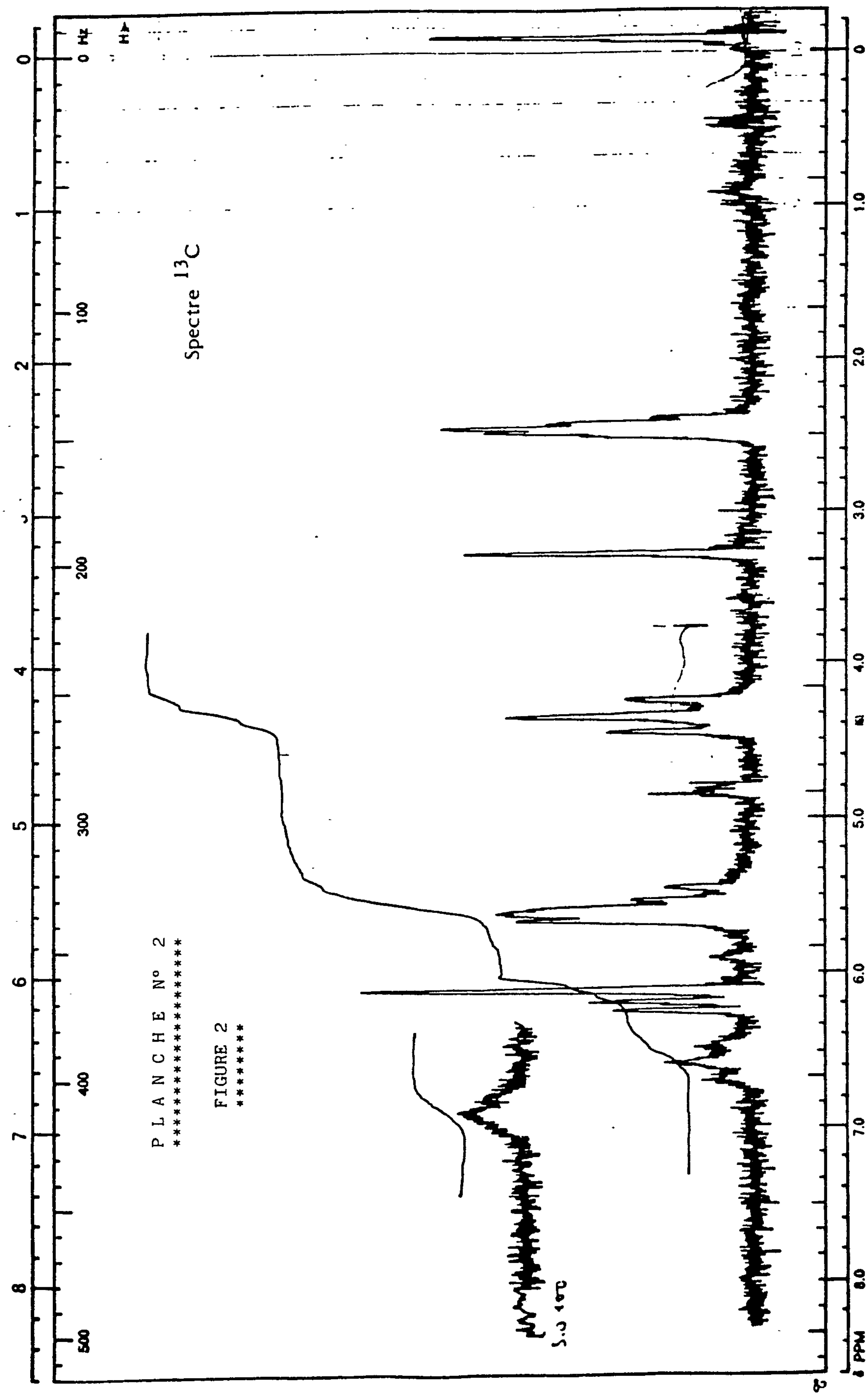
PLANCHE N° 2
FIGURE 2
Spectre 13C
0 Hz
100
200
300
400
500
0
1
2
4
5
6
7
8
0
1.0
2.0
3.0
4.0
5.0
6.0
7.0
8.0
δ PPM

PLANCHE N° 3

FIGURE 3

Spectre Proton